# EUROPEAN PATENT APPLICATION

(11) **EP 0 669 137 A1**
(43) Date of publication of application: **30.08.1995**
(21) Application number: 94200465.6
(22) Date of filing: 24.02.1994
(51) Int. Cl.: A61L 9/12

(54) **A device for vaporizing an active substance**

(71) Applicant: Sara Lee/DE N.V., NL-3532 AA Utrecht (NL)
(72) Inventor: Duterloo, Caroline G., NL-2317 DS Leiden (NL)
(74) Representative: Smulders, Theodorus A.H.J., Ir.

(57) **Abstract**

The invention relates to a device for evaporating an active substance, such as an insecticide or a perfume composition, comprising a reservoir for at least one liquid composition containing said active substance, which device comprises means for evaporating said composition and means for supplying said composition to the means for the evaporation thereof, the supply capacity of said supply means being smaller than the evaporative capacity of the means for the evaporation.

## Description

The present invention relates to a device for evaporating an active substance such as an insecticide or a perfume composition, i.e., an insecticide evaporator or an air-freshener.

A large number of air-fresheners (in this connection understood to include an insecticide evaporator) are based on the system whereby the liquid to be evaporated is supplied by means of a supply, generally a wick, to an evaporation surface, a blotter.

In these evaporators a liquid is used which consists of an active substance dissolved in a volatile organic solvent, or an active substance which has been emulsified in water by means of a suitable emulsifier. Also known are systems based on a combination of organic solvent, water and emulsifier.

An active substance as used in the present systems of insecticide evaporators or air-fresheners consists of a single substance or a combination of two of more components. Generally, the use of a single substance only occurs with insecticides, and in that case, too, it is preferred to use combinations of two or more components. Perfume compositions in practice always involve complex systems of a large number of chemical compounds.

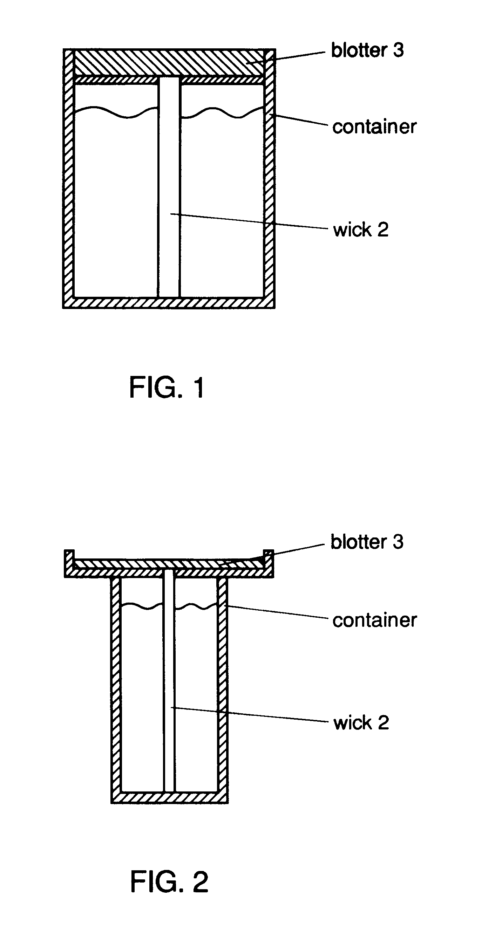

Figure 1 diagramatically shows an example of the construction of a conventional air-freshener or insecticide evaporator. It consists of a container 1, a wick 2 and a blotter 3. Contained in the container 1 is an active substance (in solution). This substance is sucked up by way of the porous wick 2 to the blotter until the latter is completely saturated and is released to the surroundings through evaporation from the surface of the blotter. Because of the continuous supply of new liquid, the evaporation rate and the composition of the released vapor are virtually constant. In the appended Graph 1 the curve of the amount of evaporated material in time is indicated.

In this system, both at the beginning of the life of the product and during its life, the amount of liquid in the reservoir is larger than the absorption capacity of the wick and the blotter.

There is an increasing need for the reduction of the amount of solvent which is introduced into the atmosphere. It is true that the use of water provides a solution to the problem of the entry of organic solvent into the atmosphere, but the action of the evaporators is thereby adversely affected to the extent where it is insufficient.

Accordingly, with regard to the use of air-fresheners and insecticide evaporators, systems are being searched for which enable working with perfume or insecticide containing substantially no organic solvent. In practice this means that systems are aimed for in which the content of active substances is at least 25, preferably at least 50, and more particularly more than 90 wt.%.

Owing to the lesser amount of solvent, the amount of liquid is much smaller. This reduction of the liquid volume may run up to a factor of 10 in the case where pure active substance without solvent is used. If the same wick and blotter as are conventional for the diluted solutions are used, the absorption capacity of the wick and the blotter will be greater than the total amount of liquid in the reservoir. The total amount of liquid will therefore be sucked up completely by the wick and the blotter.

Even apart from the fact that the liquid reservoir is now empty virtually immediately, which gives the user the impression that the life of the product is limited, a problem presents itself in that the release of the active substance is not constant in time. A logical solution to this problem would be to adjust the volume of blotter, wick and reservoir. However, it has been found that this does not lead to satisfactory results.

Experiments have demonstrated that the evaporation rate of such a system is not constant and decreases with time. Graph 1 ^{b} graphically represents the evaporation rate of such a system. It clearly shows that the intensity of the release declines very strongly. This is caused inter alia by the fact that the slowly evaporating components accumulate in the blotter and occupy a portion of the evaporation surface that increases with time. As a result, the evaporation rate is increasingly determined by the poorly evaporating components. Since the supply of the active substance to the blotter is determined by the evaporation rate, the supply decreases in time as well. As a consequence, the composition of the evaporating components is not constant either.

The present invention is based on the surprising insight that it is essential to a proper operation of an evaporator that the blotter remain unsaturated, even if there is still liquid present in the supply reservoir.

The supply capacity of the wick must therefore be smaller than the maximum evaporative capacity of the blotter, while the absorption capacity of the blotter is so large that the blotter never becomes saturated. This ensures that the suction of the active component is maintained.

The invention accordingly comprises a device for evaporating an active substance, comprising a reservoir filled with at least one liquid composition, which device comprises means for evaporating the composition (blotter) and means for supplying the composition to the means for the evaporation thereof (wick), the supply capacity of the supply means being smaller than the evaporative capacity of the means for the evaporation, while the absorption capacity of the means for the evaporation is so large that these do not become saturated.

The crux of the invention accordingly resides in the fact that the supply of the active substance to the evaporation means is limited in such a manner that the evaporation surface never becomes saturated. In this way a system is obtained which provides a homogeneous and linear evaporation of the active substance at all times. The reason is that the capillary action of the blotter is maintained, which prevents saturation with heavy components in the proximity of the supply of the components to the blotter. In fact, through this capillary action the heavy components are, as it were, sucked away from the supply. Then these heavy components do not get an opportunity to saturate the surface near the supply, so that there is always room for the supply of fresh composition, which also provides for a linear evaporation.

As for the construction of the blotter, this means that it is so large that the amount of composition to be evaporated cannot saturate the surface.

In accordance with the invention it is essential that the supply capacity to the evaporation surface is limited. This can be controlled in a number of ways, for instance through supply means of a low porosity, by the selection of a small diameter or by providing a restriction, which may or may not be controllable, in the supply means. The dosing rate is also influenced by the porosity of the blotter, the viscosity of the liquid and, if applicable, an underpressure or overpressure in the liquid reservoir.

In accordance with an embodiment of the present invention, the evaporation rate and hence the capacity of the blotter can be increased by continuously refreshing the air in the environment of the blotter. This may for instance be realized by using a fan or by arranging for the blotter itself to function as a 'fan', i.e. the blotter rotates.

The vaporizer according to the invention is suitable for evaporating insecticides or perfumes in a substantially pure form. However, it is also possible to use compositions with a lesser proportion of active substance, for instance with proportions of 10 wt.% and less.

The use of such minor proportions of active substance still provides the advantage that a highly constant and controlled release of the active substance takes place. However, the advantages of the invention are brought out to a maximum when use is made of higher concentrations of active components. In general, it is preferred to use concentrations of at least 25 wt.% active substance in the organic solvent. More particularly, it is preferred to start from solutions having a content of at least 50 wt.%. Most preferred are systems based on substantially pure active substance. This means that the amount of active substance is generally not less than 70, preferably not less than 90 and more particularly not less than 95 wt.%.

As perfume, the various suitable perfume compositions that are available for air-fresheners can be used. This type of perfume compositions can be used as such or in combination with a slight amount of solvent. Other active substances, which can be used alone or in combination with perfumes, are odor absorbents, odor modifiers, disinfectants, and substances such as mentioned in EP-A 37375 and US-A 4,663,315.

When the evaporator is used for insecticides, preferably one or more insecticides that are liquid by nature are used. However, it is also possible to dissolve these in a slight amount of solvent, but the amount of solvent must be kept as limited as possible. It is also possible to further include insect-repellent or insect-baiting substances in the system. The system according to the invention comprises a liquid reservoir, supply means and an evaporation surface, see Figure 2. The supply means should be constructed in such a manner that the desired restriction in the supply to the blotter is obtained. It is for instance possible to use, as a supply, a system based on a capillary or on a porous material which are in direct contact with the blotter. Suitable capillaries are made from glass, metal or synthetic material. By varying the number and the diameter of the capillary, the dosing to the blotter can be controlled. This may be advantageous if it is desired to make the magnitude of release adjustable.

If, as wick, a porous material is used, use can be made of paper, porous synthetic material, porous ceramic material, porous metal, porous glass, wood, nonwoven, glass fibers and the like. With these materials, too, it is possible to adjust the magnitude of the dosing. This can be effected by providing an adjustable restriction around the wick, or by providing a plurality of wicks which can be activated and deactivated in a suitable manner.

A third system for dosing may be based on a drip system, whereby the active substance is dripped onto the evaporation surface. In this connection, see European patent application No. 78114.

It is also possible to use a different form of driving force for dosing, for instance a certain degree of overpressure in the container, which is obtained through osmotic action, as described in Applicant's European patent application No. 93201166, or in a different manner.

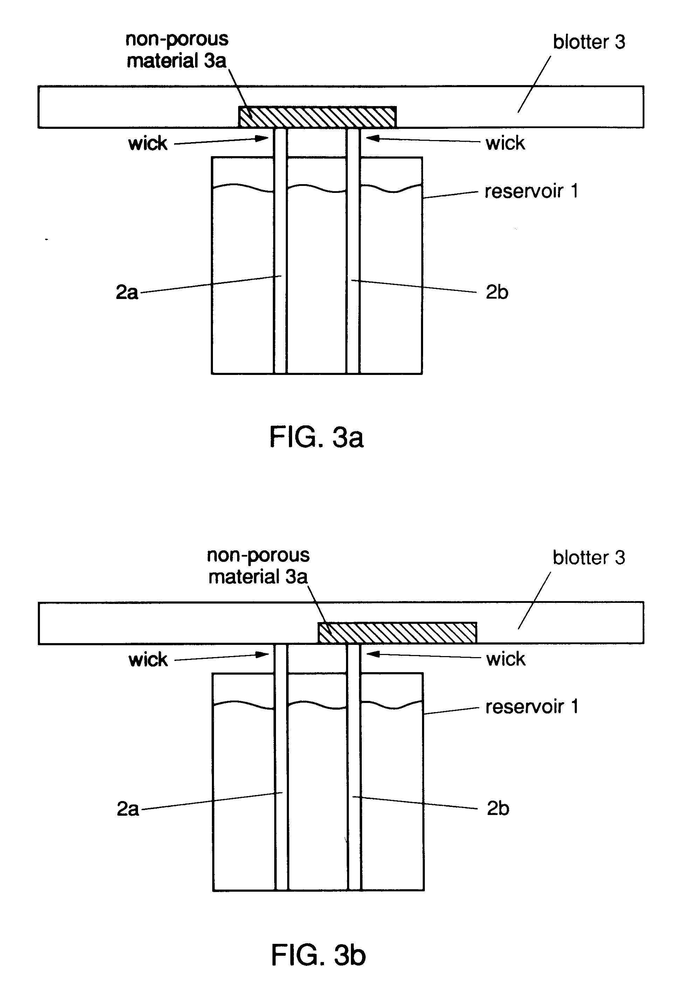

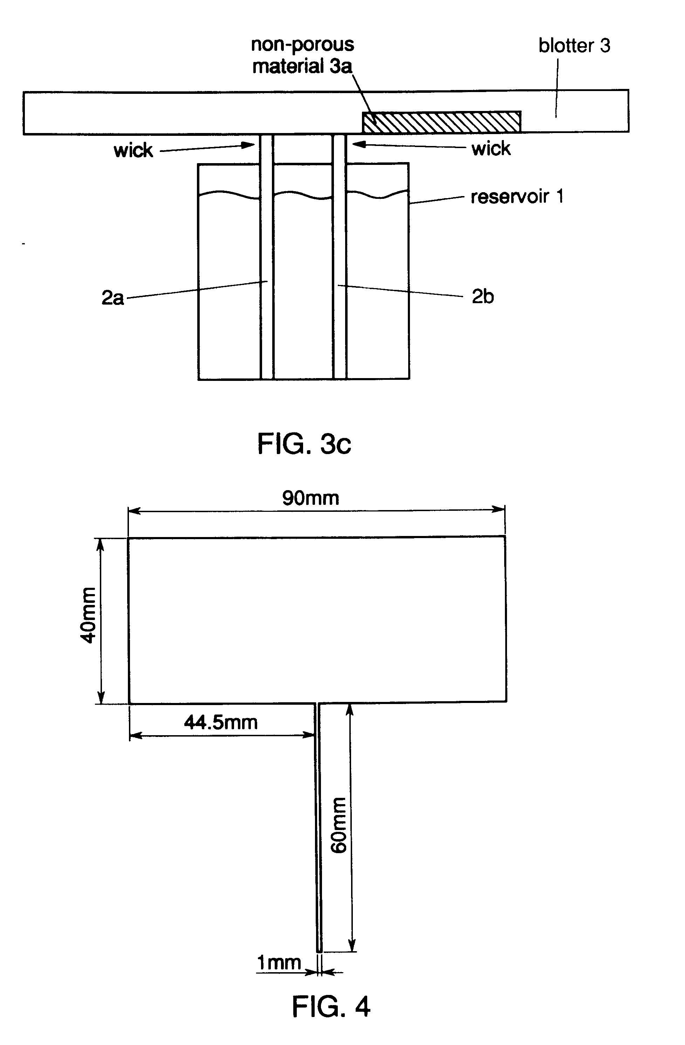

As already indicated above, the dosing of the active substance can be controlled by providing a suitable construction. An example thereof is included in Fig. 3. This drawing shows a device in which a container 1 comprises two dosing means 2a, 2^{b}, which touch a blotter 3. This blotter comprises of a non-porous part 3a. If the dosing means are exclusively in contact with this non-porous part, the evaporator is not in operation, at any rate no active substance is sucked up. In Fig. 3^{b} one half of the dosing means is in contact with the non-porous part and the other half with the porous part. In this position the dosing amounts to half of its maximum. In the position shown in Fig. 3° the means are completely in contact with the porous part of the blotter so that the evaporator is fully in operation. The actual construction of an evaporator working according to this principle is simple for a person of ordinary skill in the art. It is for instance possible to use a rotatable blotter or a slidable system. Naturally, this idea can also be implemented in different ways, for instance by using a system in which the dosing means are height- adjustable.

The invention is now elucidated on the basis of an example which should not be seen as a limitation.

### Example 1

A paper porous blotter material (Binzer Germany, quality KS 617-650 g/m²) 90 x 40 mm was laminated to a nonwoven (Crompton England, 19 g/m², ref. 784). A wick, 60 mm long and 1 mm wide, was formed on the nonwoven by cutting (see Fig. 4).

The blotter material and the nonwoven were laminated together by stitching them onto each other with a cotton thread along the edges of the blotter material.

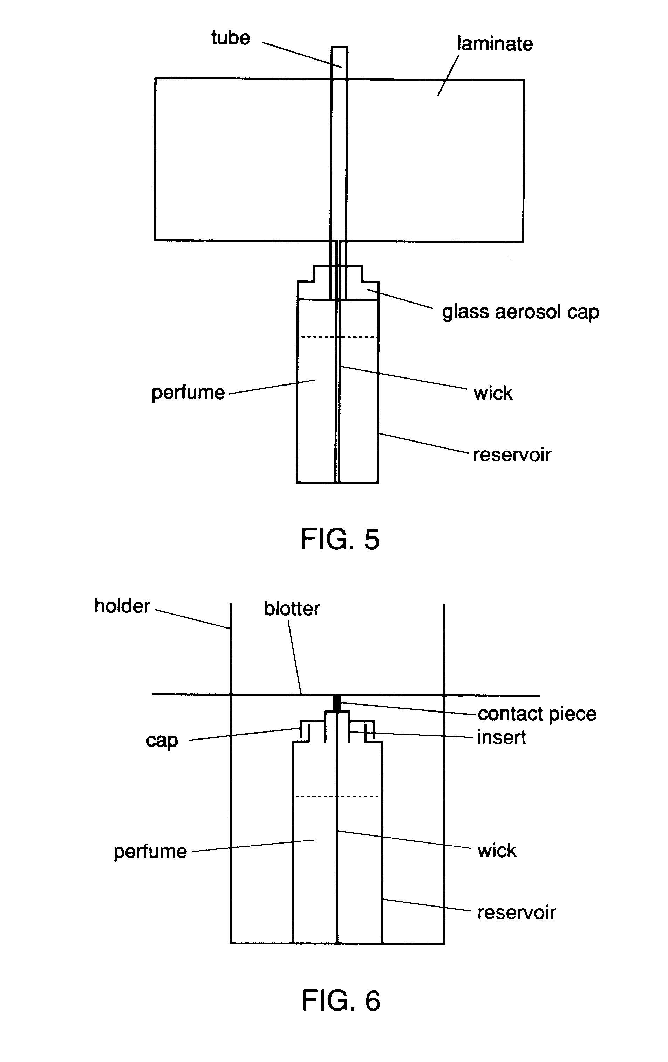

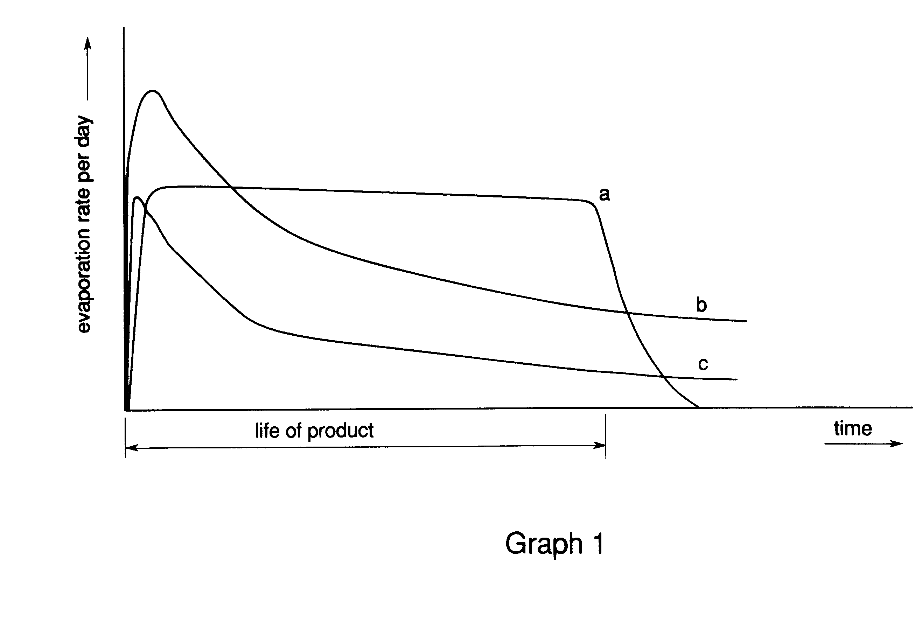

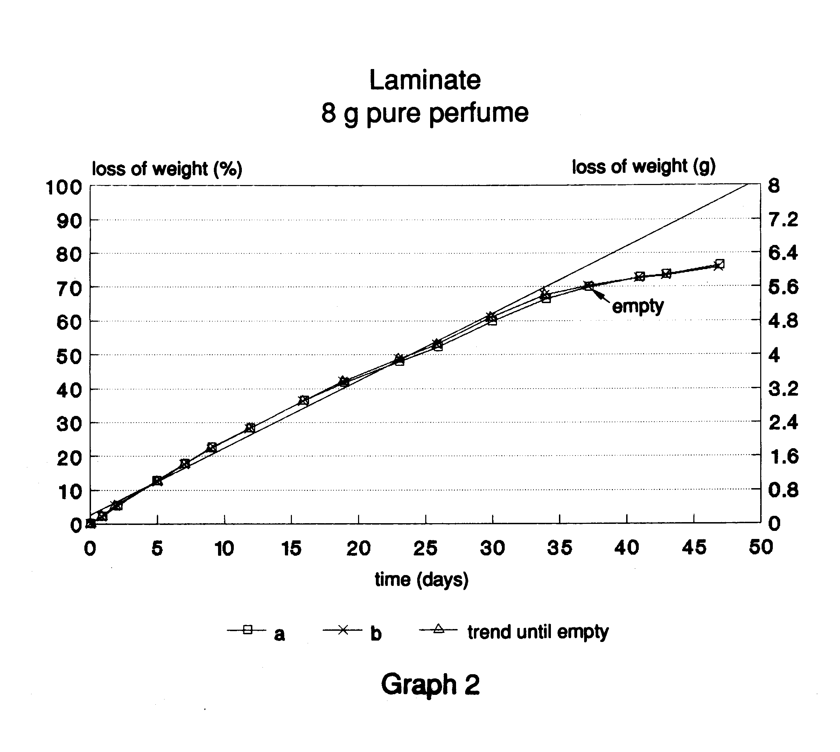

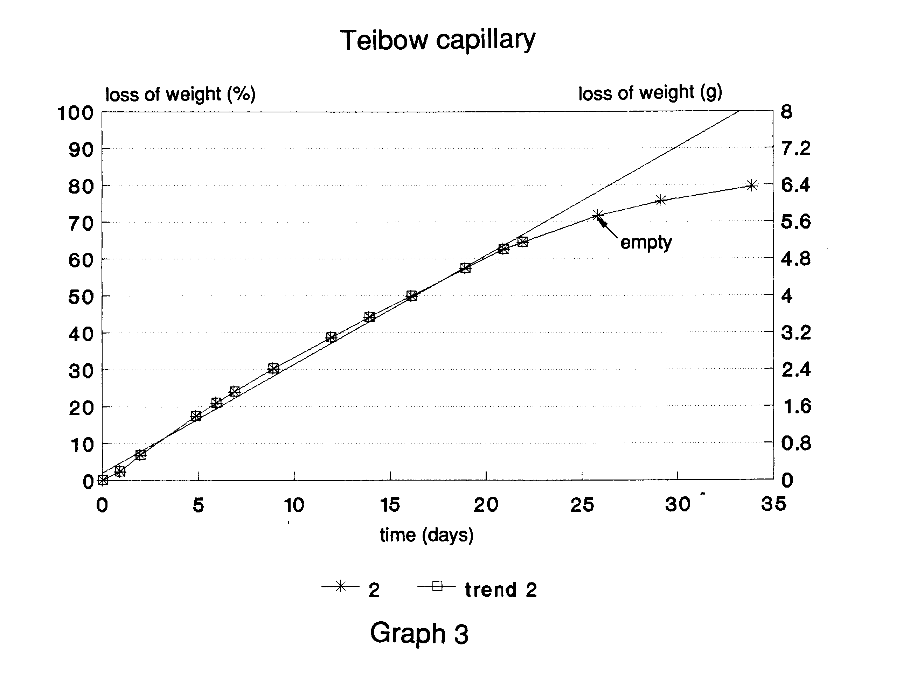

A plastic tube of a length of 75 mm was cut lengthwise over 50 mm. The blotter was fitted between the two halves, with the wick being inserted through the uncut portion of the tube. This assembly, by way of the wick and the tube, was placed in a bottle, in such a manner that the orientation of the laminate was properly vertical (see Fig. 5).

The bottle was filled with 8 g pure perfume (Lucta Myralda AE-22411 P), so without the addition of solvents.

The loss of weight through evaporation as a function of time was determined, see Graph 2. The graph shows that at a loss of weight of 70% or 5.6 g, 100% of the perfume has been dosed on the blotter: the reservoir is empty after more than 35 days.

Up to the time when the reservoir is empty, the loss of weight through evaporation is linear: 0.16 g/day. Once the reservoir is empty, the evaporation decreases strongly. The life of the product coincides with the presence of perfume in the reservoir.

### Example 2

A capillary (Teibow C-3D 0.38) was inserted into a piece of porous material (Filtrona, TPU 3550) which is in communication with a paper blotter (Binzer 183, 210 g/m²) of 100 x 100 mm.

The capillary was placed in a reservoir with 8 g perfume (Givaudan, Pino nouvea F). To ensure that the contact between the blotter and the contact piece remains good, the assembly was placed in a holder (see Fig. 6).

The evaporation of the perfume in time is shown in Graph 3 by plotting the loss of weight (in % and grams) against time.

The graph shows that at a loss of weight of 70% or 5.6 g, 100% of the perfume has been dosed on the blotter: the reservoir is empty after 25 days.

Up to the time when the reservoir is empty, the loss of weight through evaporation is linear: 0.23 g/day. Once the reservoir is empty, the evaporation decreases strongly. The life of the product accordingly coincides with the presence of perfume in the reservoir.

## Claims

1. A device for evaporating an active substance, such as an insecticide or a perfume composition, comprising a reservoir for at least one liquid composition containing said active substance, which device comprises means for evaporating said composition and means for supplying said composition to the means for the evaporation thereof, the supply capacity of said supply means being smaller than the evaporative capacity of the means for the evaporation.

2. A device according to claim 1, wherein the absorption capacity of the means for the evaporation is so large that they do not become saturated with liquid.

3. A device according to claim 1 or 2, wherein it contains a liquid composition, which composition contains an organic solvent and an active component.

4. A device according to claim 3, wherein the liquid composition contains at least 25 wt.%, preferably at least 50 wt.% active substance.

5. A device according to claim 3 or 4, wherein the liquid composition contains substantially pure active substance as defined herein.

6. A device according to claims 1-5, wherein as active substance an insecticide or a perfume composition is used.

7. A device according to claims 1-6, wherein means are present for controlling the dosing rate of the liquid composition to the means for the evaporation thereof.
